(19) Europäisches Patentamt

European Patent Office

Office européen des brevets

(11) **EP 0 938 342 B1**

(12) **EUROPÄISCHE PATENTSCHRIFT**

(45) Veröffentlichungstag und Bekanntmachung des Hinweises auf die Patenterteilung:
**10.03.2004   Patentblatt 2004/11**

(21) Anmeldenummer: 97952720.7

(22) Anmeldetag: **03.12.1997**

(51) Int Cl.[7]: **A61K 47/48**, A61K 9/00

(86) Internationale Anmeldenummer:
**PCT/DE1997/002903**

(87) Internationale Veröffentlichungsnummer:
**WO 1998/030245 (16.07.1998 Gazette 1998/28)**

(54) **INJIZIERBARE POLYMERZUSAMMENSETZUNG ZUR HERSTELLUNG EINES IN-SITU-IMPLANTATS**

INJECTABLE COMBINATION OF POLYMERS FOR THE MANUFACTURE OF AN IN SITU IMPLANT

COMBINAISON INJECTABLE DE POLYMERES POUR L'OBTENTION D'UN IMPLANT IN SITU

(84) Benannte Vertragsstaaten:
**AT BE CH DE DK ES FI FR GB GR IE IT LI LU MC NL PT SE**

(30) Priorität: **10.01.1997  DE 19701912**

(43) Veröffentlichungstag der Anmeldung:
**01.09.1999   Patentblatt 1999/35**

(73) Patentinhaber: **Schering Aktiengesellschaft 13353 Berlin (DE)**

(72) Erfinder:
 • **DITTGEN, Michael**
   **D-99510 Apolda (DE)**
 • **FRICKE, Sabine**
   **D-07749 Jena (DE)**
 • **GERECKE, Hagen**
   **D-07743 Jena (DE)**
 • **MÖLLER, Ines-Patricia**
   **D-07743 Jena (DE)**
 • **VÖLKEL, Christoph**
   **D-07743 Jena (DE)**

(74) Vertreter: **Cramer, Eva-Maria Jenapharm GmbH & Co.KG, Patentabteilung, Otto-Schott-Strasse 15 07745 Jena (DE)**

(56) Entgegenhaltungen:
WO-A-86/00533          WO-A-88/04557
WO-A-90/03768          WO-A-95/35093
DE-C- 19 701 912

 • JEONG B. ET AL: "Biodegradable block copolymers as injectable drug-delivery systems" NATURE, 1997, 388/6645 (860-862), UNITED KINGDOM, XP002068338
 • MIYAMOTO S ET AL: "Polylactic acid-polyethylene glycol block copolymer. A new biodegradable synthetic carrier for bone morphogenetic protein." CLIN ORTHOP, SEP 1993, (294) P333-43, UNITED STATES, XP000600897
 • HA J -H ET AL: "Albumin release from bioerodible hydrogels based on semi-interpenetrating polymer networks composed of poly(epsilon -caprolactone) and poly(ethylene glycol) macromer" JOURNAL OF CONTROLLED RELEASE, Bd. 49, Nr. 2-3, Dezember 1997, Seite 253-262 XP004101951
 • SCHMIDMAIER G ET AL: "A new biodegradable polylactic acid coronary stent-coating, releasing PEG-Hirudin and a prostacycline analog, reduces both platelet activation and plasmatic coagulation" JOURNAL OF THE AMERICAN COLLEGE OF CARDIOLOGY, 29 (2 SUPPL. A). 1997. 354A., XP002068341
 • VAN DER GIESSEN W.J. ET AL: "Marked inflammatory sequelae to implantation of biodegradable and nonbiodegradable polymers in porcine coronary arteries" CIRCULATION, 1996, 94/7 (1690-1697), USA, XP002068343
 • BHARDWAJ R ET AL: "In vitro evaluation of Poly(d,l-lactide-co-glycolide) polymer -based implants containing the alpha-melanocyte stimulating hormone analog, Melanotan-I" JOURNAL OF CONTROLLED RELEASE, Bd. 45, Nr. 1, 3.März 1997, Seite 49-55 XP004099354

**Beschreibung**

Die vorliegende Erfindung betrifft Polymerzusammensetzungen zur Herstellung eines In-situ-Implantates. Hierbei wird ein steriler spritzbarer wasserunlöslicher Komplex aus einem bioabbaubaren Polymer und einem biokompatiblen endgruppenfunktionalisierten Polyether im Organismus plaziert, der unter Einfluß der Körperflüssigkeiten koaguliert.

Bekannte Implantate zur Applikation von biologisch aktiven Stoffen bestehen aus Mikropartikeln, oder sie werden durch Verpressung unter aseptischen Bedingungen hergestellt. Beispiel für ein gepreßtes Implantat ist das im Handel befindliche Präparat Zodalex®. Dieses Implantat ruft am Ort der Applikation einen erheblichen Schmerz hervor. Ähnliche Nachteile treten bei den in Form von Mikropartikeln injizierten Präparaten Parlodel®, Profact® Depot, Enantone®-Gyn Monatsdepot und Decapeptyl Gyn auf.

Das gebräuchlichste Herstellungsverfahren für Mikropartikel bzw. Microspheren ist die "Solvent Evaporation Technique", wobei organische und für den lebenden Organismus toxische Lösemittel verwendet werden.

Bei der "Solvent Evaporation Technique" wird eine Emulsion des bioabbaubaren Polymers verwendet, aus der das Lösemittel schrittweise entfernt wird. Die geeigneten Lösemittel, wie Methlyenchlorid [Hora, M.S. et al., Bio/Technology 8(1990), 755 - 758; Bodmeier, R. et al., Pharm. Res. 12 (8/1995), 1211 - 1217; Lu, W.Q. et al., Biotechn. Prog. 11 (2/1995), 224 - 227; Cohen, S. et al., Pharm. Res. 6 (8/1991), 713 - 720], Mischungen von Methylenchlorid und Methanol [Metha, R.C. et al., J.

Contr. Rel. 29 (1994), 375 - 384], Dichlormethan [Kissel, T. et al., J. Contr. Rel. 39 (1996), 315- 326] oder Chloroform [Hayashi, Y. et al., Pharm. Res. 11 (2/1994), 337 - 340] sind physiologisch zumindest bedenklich. Auch bei der "spray Drying Method" wird das bioabbaubare Polymer in Lösemitteln wie z.B. Methylenchlorid [Bodmeier, R. et al., Pharm. Res. 12 (8/1995), 1211 - 1217] gelöst und anschließend in einem Sprühvorgang mit der biologisch aktiven Substanz zu der festen Form verarbeitet. Ein gemeinsamer Nachteil beider Herstellungsmethoden von Mikropartikeln ist der Restlösemittelgehalt. Die "spray Drying Method" erfordert darüber hinaus einen relativ hohen apparativen Aufwand.

In US-4,938,763 wird die Herstellung in-situ geformter Implantate beschrieben. Bei diesem Herstellungsverfahren werden bioabbaubare Polymere, z.B. Polylactide und Polylactid-co-glycolide, in einem Lösemittel gelöst. Die Lösung wird injiziert. Bei Kontakt mit der Körperflüssigkeit bildet sich ein festes Implantat, bestehend aus dem gefällten bioabbaubaren Polymer und der biologisch aktiven Substanz aus, in dem das Lösemittel aus dem Implantat vollständig herauswandert und sich im Organismus verteilt ("dissipate"). Als Lösemittel werden Ethanol, Propylenglycol, Ketone u.a. genannt. Nachteil dieser Methode ist allerdings, daß die aufgeführten Lösemittel physiologisch wirksam und daher nicht oder nur in geringem Umfang parenteral applizierbar sind. Desweiteren wird ein Verfahren zur Herstellung in situgeformter Implantate beschrieben, wonach eine Lösung von Monomeren oder Oligomeren ("prepolymers") mit Beimischung von Startersubstanzen und/oder Katalysatoren injiziert wird. Nach der Injektion polymerisieren die Bestandteile zu einem bioabbaubaren Polymer. Bei diesem Verfahren ist der Einsatz hochreaktiver Substanzen als Starter zur Polymerisation erforderlich, wobei diese toxisch sind.

Nach Eliaz et al. [Proc. 3rd Jerusalem Conference on Pharmaceutical Sciences and Clinical Pharmacology, Sept. 1 - 6, 1996] kann nach der gleichen Methode ein in-situ geformtes Implantat hergestellt werden, wenn Glycofurol als niedermolekulares Lösemittel für Homo- und Copolymere von Polymilchsäure verwendet wird. Die so hergestellten Implantate eignen sich besonders für Proteine. Die proteinhaltigen Lösungen sind niederviskos und daher gut spritzbar. Glycofurol ist mit Wasser mischbar und pharmazeutisch relativ unbedenklich.

Während der In-situ-Formung flutet Glycofurol sofort mit der wäßrigen Umgebung ab. Dadurch kann die Freisetzung des Wirkstoffs aus dem Implantat nachteilig beeinflußt werden. Untersuchungen haben dieses Ergebnis bestätigt.

In US-3,887,699 wird ein Verfahren zur Herstellung von subkutan applizierbaren Implantaten beschrieben, das auf der Bildung geformter Kugeln auf der Basis von Polymilchsäure-Homopolymeren bzw. Copolymeren mit Glykolsäure mit ausreichend hohem Molekulargewicht und einem Wirkstoff beruht. Die Integrität der hergestellten Kugeln bleibt nach Implantation über einem langen Zeitraum erhalten, und der Wirkstoff wird verzögert freigesetzt. Als geeignete Wirkstoffe werden kontrazeptiv wirksame Steroide genannt.

Ein wesentlicher Nachteil dieser wirkstoffhaltigen Partikeln ist die Herstellung mit Hilfe der "solvent Evaporation Method" und organischen Lösemitteln (Chloroform). Auf die Gefahr, die von Resten des Lösemittels ausgeht, wurde oben hingewiesen.

Eine ähnliche Methode wird in US 3,773,919 beschrieben. Demnach können pharmazeutische Depotformulierungen, die auf parenteralem Wege ausreichende Mengen Wirkstoff kontrolliert freisetzen, leicht hergestellt werden, wenn das Wirkstoffdepot größer ist als eine Einzeldosis und eine homogene Mischung aus Polymilchsäure und Wirkstoff in einem Verhältnis von jeweils 1 bis 99 vorliegt. Der Wirkstoff sollte in diesem Fall eine endokrin wirksame oder fertilitätskontrollierende Substanz sein.

Nachteil dieser Methode ist, daß die festen Partikeln in einer Salzlösung oder in einem pharmazeutisch geeigneten Öl suspendiert werden müssen, bevor sie injiziert werden können. Dabei muß mit Homogenitätsproblemen dieser Suspensionen gerechnet werden.

In WO-9517901 wird eine cytostatische Komposition beschrieben, die "intralesional" injizierbar ist. Diese enthält das Cytostatikum in einer Matrix aus einer nicht mit Wasser mischbaren Fettsäure.

Diese Komposition hat den Vorteil, daß sie soweit fließfähig ist, daß sie injiziert werden kann. Aber die Verlängerung der Wirkung des Cytostatikums wird durch die Viskosität der Matrix bewirkt. Die Viskosität der Matrix ist jedoch ein unzuverlässiger Parameter, da sie je nach vorhandener Körperflüssigkeit schwanken kann. Außerdem ist bekannt, daß Fettsäuren nur eingeschränkt biokompatibel sind und das Gewebe schädigen können.

In WO-9103491 ist eine Matrix auf Proteinbasis beschrieben, die die Zellproliferation effektiv hemmen soll. Sie enthält entweder ein Collagen oder ein Fibrinogen.

Diese Matrix erfordert geringere Mengen von Protein gegenüber herkömmlichen Systemen. Nachteile dieser Erfindung sind, daß die Freigabe des Wirkstoffs nicht durch die Zusammensetzung der Matrix gesteuert werden kann und daß zur Retardierung ein Vasokonstriktor vonnöten ist.

In EP-0341007 wird auf ein Verfahren zur Bildung eines in situ geformten Adhesivs verwiesen, das durch Kontakt des collagenhaltigen Agens mit dem Wundplasma koaguliert.

Vorteil des Verfahrens ist die sofortige Wirkungsentfaltung nach Applikation durch Bildung des Koagulats, das zum Wundverschluß führt. Diese Technik ist allerdings nur für dermale Präparationen während und nach chirurgischen Eingriffen anwendbar und schließt die Einarbeitung von Wirkstoffen nicht ein.

In EP-0328389 wird eine Methode zur Injektion einer makromolekularen Matrix mit Vinca-Alkaloiden zur Behandlung intrakranialer Tumoren beschrieben.

Die makromolekulare Matrix auf Proteinbasis fördert die Wanderung des Alkaloids weg von der Verletzung und so, daß lokal hohe Wirkspiegel erzielt werden. Nachteilig ist aber, daß für diesen Effekt ein Vasodilator zugesetzt werden muß, so daß die Steuerung der lokalen Wirkung problematisch bleibt.

Eine ähnliche Situation liegt bei WO-8902734 vor. Diese Erfindung beschäftigt sich mit einem pharmazeutischen Vehikel, daß Vasokonstriktoren und zytotoxisch wirksame Substanzen zur Behandlung neoplastischer Verletzung appliziert.

Es sind aber in diesem Vehikel Modifikatoren nötig, die die Zellstruktur modifizieren, um die Zellpermeabilität zu verändern.

Nach US-4,619,913 kann eine Collagen- oder Fibrinogenhaltige Matrix hergestellt werden, die in einem wäßrigen Medium eine amorphe fließfähige Masse bildet, die es ermöglicht, neoplastische Verletzungen oder das sie umgebende Gewebe zu behandeln.

Durch diese Technologie soll der Abtransport des Wirkstoffs in unerwünschte Regionen verhindert werden und ein erhöhter therapeutischer Effekt erzielt werden. Gleichzeitig wird der Wirkstoff gegen metabolische Inaktivierung geschützt. Diese Technik bietet eindeutige Vorteile bei Tumoren, die mit chirurgischen und radiotherapeutischen Mitteln nicht behandelbar sind. Collagene sind jedoch immunologisch nicht unbedenklich und können zu unerwünschten Reaktionen des Organismus führen.

In EP-167263 wird die gleiche Methode zur Herstellung einer Polymermatrix beschrieben, die einen zytotoxischen Wirkstoff enthält und diesen unter Verhinderung unerwünschter systemischer Effekte langsam freisetzt.

Diese Technik verringert in hohem Maße zytotoxische Nebenwirkungen. Auch hier ist die Applikation mit der Gabe eines Vasokonstriktors verbunden, der das Abfluten des Wirkstoffs über die Blut- oder Lymphbahn verhindern soll. Auf dessen Nachteile wurde schon hingewiesen.

In US-5290552 wird eine adhesive Matrix beschrieben, die vorwiegend zum Verschluß von Wunden nach chirurgischen Eingriffen verwendet werden soll. Sie koaguliert im pH-Bereich um 5 und ist daher für die Behandlung dermaler Verletzungen gut geeignet.

Für die parenterale Applikation ist diese Matrix aber ungeeignet, da pH-Werte der Körperflüssigkeiten die Matrix nicht zur gewünschten Koagulation bringen.

Miyamoto et al. (Miyamoto, S. et al., Clin. Orthop. 1993 (294), S. 333-43) beschreiben ein In-situ-Implantat zur Freisetzung des Hormons BMP, das durch die Injektion eines Polyacetat-Polyethylenglykol-Blockpolymer-Komlpexes erhalten wird. Hierbei soll das Blockpolymer den aktiven Stoff so schnell wie möglich abgeben, um eine Knochenbildung zu induzieren und unerwünschte Nebenwirkungen zu verhindern.

WO-95/53093 beschreibt die Bildung eines In-situ-Implantates zur Freisetzung verschiedener biologisch aktiver Stoffe durch die Injektion eines Komplexes aus Polymethacrylsäure und Polyethylenglykol. Die aufgeführten Polymethacrylate sind jedoch nicht bioabbaubar.

Der vorliegenden Erfindung liegt die Aufgabe zugrunde, einen injizierbaren Komplex zur Herstellung eines In-situ-Implantates zur Verfügung zustellen, der die Nachteile des Standes der Technik überwindet.

Die Aufgabe wird erfindungsgemäß dadurch gelöst, daß ein steriler spritzbarer wasserunlöslicher Komplexe aus einem bioabbaubaren Polymer und einem biokompatiblen endgruppenfunktionalisierten Polyether injiziert wird. Dieser koaguliert unter dem Einfluß der Körperflüssigkeit und bildet so ein In-situ-Implantat.

Bei einer Untersuchung einer großen Zahl von biokompatiblen Komplexbildnern wurde überraschenderweise gefunden, daß endgruppenfunktionalisierte Polyether mit definierten Strukturen in einem engem Konzentrationsbereich in der Lage sind, mit bioabbaubaren Polymere aus der Gruppe der Poly($\alpha$-hydroxy)ester und deren Copolymeren sterile spritzbare wasserunlösliche Komplexe zu bilden.

Bevorzugt sind daher Komplexe zur Herstellung von Insitu-Implantaten, bei denen das bioabbaubare Polymer eine Verbindung aus der Gruppe der Poly($\alpha$-hydroxy)ester oder deren Copolymeren ist.

Bevorzugt sind ferner Komplexe, bei denen der biokompatible Polyether eine Verbindung der allgemeinen Formel I

$$\text{R-CH}_2\text{-[CH}_2\text{-O-CH}_2]_n\text{-CH}_2\text{-R} \qquad \text{(I)}$$

ist, wobei

R für -OH, -CH$_2$OH oder CH$_2$CH$_2$OH steht,
n eine ganze Zahl von 4 bis 12 bedeutet,
und die Molmasse der Verbindung der Formel I 200 bis 600 Masseneinheiten beträgt.

Besonders bevorzugt sind Komplexe zur Herstellung von Insitu-Implantaten bei denen R für -OH steht.

Bevorzugte Komplexe weisen ein Verhältnis zwischen bioabbaubarem Polymer und Polyether zwischen 1: 100 und 1:2, insbesondere bevorzugt zwischen 1:6 und 1:3, auf.

Die erfindungsgemäßen Komplexe enthalten somit nur die oben genannten biokmpatiblen Polymere und kommen ohne Zusatz von Startersubstanzen oder Katalysatoren zur Anwendung.

Die erfindungsgemäßen Komplexe können biologisch aktive Stoffe enthalten. Bei bevorzugten Komplexen enthält das Koagulat mindestens einen biologisch aktiven Stoff, beispielsweise aus der Gruppe der Hormone, Immunmodulatoren, Immunsuppressiva, Antibiotika, Zytostatika, Diuretika, Magen-Darm-Mittel, Herz-Kreislauf-Mittel, Antiinflammatoria, Analgetika, Lokalanaesthetika und/oder Neuropharmaka.

Die geeigneten endgruppenfunktionalisierten Polyether haben die allgemeine Formel I

$$\text{R-CH}_2\text{-[CH}_2\text{-O-CH}_2]_n\text{-CH}_2\text{-R} \qquad \text{(I)}$$

mit R = OH, CH$_2$OH und CH$_2$CH$_2$OH, vorzugsweise sind Polyether mit R = OH, M = 200 ... 600 und n = 4 ... 12 geeignet. Diese werden nachfolgend wie in Tab.1 angegeben bezeichnet.

Tab. 1:

| Abkürzungen der endgruppenfunktionalisierten Polyether | |
|---|---|
| PE 1 | Polyether mit R = -OH, M = 200, n = 4 |
| PE 2 | Polyether mit R = -OH, M = 400, n = 8 |
| PE 3 | Polyether mit R = -OH, M = 600, n = 12 |

Die gewünschten sterilen spritzbaren wasserunlöslichen Komplexe bilden sich jedoch nur bei geeigneten Konzentrationsverhältnissen von bioabbaubarem Polymer und endgruppenfunktionalisiertem Polyether aus (Tab. 2).

Tab. 2: Spritzbarkeit/Koagulatbildung der Komplexe bioabbaubares Polymer/PE

| bioabbaubares Polymer | PE | Konzentration bioabbaubares Polymer | | | | | | | | |
|---|---|---|---|---|---|---|---|---|---|---|
| | | 0 % | 1 % | 5 % | 10 % | 15 % | 20 % | 25 % | 30 % | 35 % |
| RG 503 | PE 1 | +/- | +/+ | +/+ | +/+ | - | - | - | - | - |
| | PE 2 | +/- | +/+ | +/+ | +/+ | +/+ | +/+ | +/+ | +/+ | - |
| | PE 3 | +/- | +/+ | +/+ | +/+ | +/+ | +/+ | - | - | - |
| RG 503 H | PE 1 | +/- | +/+ | +/+ | +/+ | - | - | - | - | - |
| | PE 2 | +/- | +/+ | +/+ | +/+ | +/+ | +/+ | - | - | - |
| | PE 3 | +/- | +/+ | +/+ | +/+ | +/+ | +/+ | - | - | - |
| RG 752 | PE 1 | +/- | +/+ | +/+ | +/+ | - | - | - | - | - |
| | PE 2 | +/- | +/+ | +/+ | +/+ | +/+ | +/+ | - | - | - |
| | PE 3 | +/- | +/+ | +/+ | +/+ | +/+ | +/+ | - | - | - |
| RG 858 | PE 1 | +/- | +/+ | +/+ | +/+ | +/+ | +/+ | - | - | - |
| | PE 2 | +/- | +/+ | +/+ | +/+ | +/+ | +/+ | - | - | - |
| | PE 3 | +/- | +/+ | +/+ | +/+ | +/+ | +/+ | - | - | - |
| L 104 | PE 1 | +/- | +/+ | +/+ | +/+ | +/+ | +/+ | - | - | - |
| | PE 2 | +/- | +/+ | +/+ | +/+ | +/+ | +/+ | - | - | - |
| | PE 3 | +/- | +/+ | +/+ | +/+ | +/+ | +/+ | - | - | - |

Legende:

| | | |
|---|---|---|
| PE | = | endgruppenfunktionalisierter Polyether |
| +/- | = | spritzbar/ kein Koagulat |
| +/+ | = | spritzbar/ Koagulat |
| - | = | nicht spritzbar |
| RG, L | = | Polylactid-co-glycolid Boehringer Ingelheim |

Bei dem Polylactid-co-glycolid RG 503 liegt der optimale Konzentrationsbereich für PE 1 zwischen 1 und 10 %, für PE 2 zwischen 1 und 30 % und für PE 3 zwischen 1 und 20 %. Bei den Polylactid-co-glycoliden RG 503 H und RG 752 liegt der optimale Konzentrationsbereich für PE 1 zwischen 1 und 10 %, für PE 2 und PE 3 jeweils zwischen 1 und 20 %. Bei dem Polylactid-co-glycolid RG 858 und bei dem Polylactid L 104 liegt der optimale Konzentrationsbereich für PE 1,2 und 3 jeweils zwischen 1 und 20 %.

Bei den voranstehend als optimal bezeichneten Konzentrationsbereichen sind die Komplexe soweit fließbar, daß sie weitgehend schmerzfrei injiziert werden können. Die Komplexe sind herkömmlichen Sterilisationsverfahren, insbesondere thermischen Verfahren, zugänglich. Sie können biologisch aktive Stoffe aus der Gruppe der Hormone, Immunmodulatoren, Immunsuppressiva, Antibiotka, Zytostatika, Diuretika, Magen-Darm-Mittel, Herz-Kreislauf-Mittel, Antiinflammatoria, Analgetika, Lokalanaesthetika und Neuropharmaka aufnehmen.

Wirkstoffe, die für diese Anwendungen in Frage kommen, sind beispielsweise Testosteron und seine Ester, Estradiol, Progesteron, Gonadoliberin-Analoga, Prostaglandine und deren Derivate, Cyclosporin, Cortison, Prednisolon, Dexamethason, Penicillin-Derivate, Cephalosporin-Derivate, Makrolid-Antibiotika, Polypeptid-Antibiotika, Proteine der Interleukin- und Interferon-Gruppe, Aciclovir, Cyclophosphamid, Methotrexat, Zidovudin, Misoprostol, Furosemid, Amilorid, Nitroglycerin, Nifedipin, Verapamil, Haloperidol, Amitryptilin, Piroxicam, Ibuprofen, Indomethacin, Diclofenac, Morphin, Pethidin, Naloxon, Tetracain, Lidocain.

Nach der Plazierung im Organismus bildet sich unter Einfluß der Körperflüssigkeit ein Koagulat. Einherschreitend mit dem biologischen Abbau dieses Koagulats, der zwischen Wochen und Monaten (RG-Reihe, Herstellerangabe Boehringer Ingelheim) und Monaten bis Jahren (L-Reihe, Herstellerangabe Boehringer Ingelheim) betragen kann, wird der enthaltene biologisch aktive Stoff freigesetzt.

Es ist daher bevorzugt, daß man die Freisetzung des biologisch aktiven Stoffes durch die Bestandteile des sterilen spritzbaren wasserunlöslichen Komplexes steuert. Dadurch ist es möglich, die Freisetzungsgeschwindigkeit den pharmokinetischen und pharmokodynamischen Eigenschaften der Wirkstoffe im Organismus anzupassen.

Vorzugsweise werden die spritzbaren Komplexe zur Bildung der Implantate sterilisiert. Diese Sterilisation erfolgt beispielsweise durch ein bekanntes thermisches Verfahren oder durch aseptische Arbeitsweise oder durch eine Kombination solcher Verfahren.

Die Erfindung wird durch die nachfolgenden Beispiele näher erläutert.

Untersuchungsmethode :

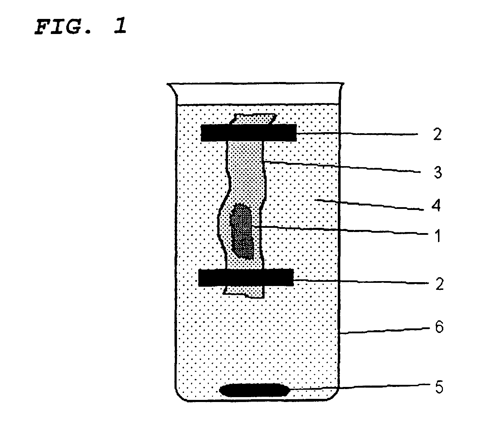

Die Freisetzung des biologisch aktiven Stoffs (Testosteron, Progesteron, Interleukin-2) wurde in einer Freisetzungsapparatur (Fig. 1) untersucht. Zu Beginn des Versuchs wurden 2 ml des jeweiligen Komplexes in einen Dialyseschlauch mit der definierten Molmassenausschlußgrenze von 100000 Dalton gespritzt. De Dialyseschlauch befand sich, oben und unten mit Verschlußklammern verschlossen, in 1000 ml isotonischer Natriumchlorid-Lösung (Akzeptormedium), die auf 37 °C temperiert und mit definierter Rührgeschwindigkeit (300 U/min.) bewegt wurde.

Beispiel 1:

| Testosteron-Komplex | |
|---|---|
| Komplex 1: | |
| Testosteron | 1 Teil |
| Resomer RG 503 | 1 Teil |
| PE 2 | 8 Teile |
| Vergleich der Freisetzung gegen eine Suspension (Referenz) folgender Zusammensetzung: | |
| Testosteron | 1 Teil |
| Erdnußöl | 9 Teile |

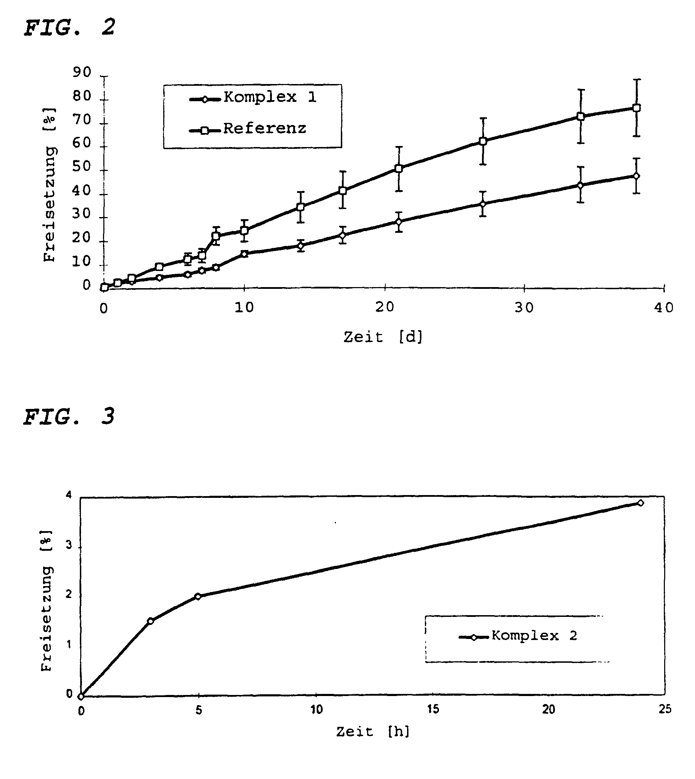

Fig. 2 zeigt die Abhängigkeit der prozentualen Freisetzung von Testosteron von der Zeit. Bei dem Vergleich der Freisetzung des Testosteron aus dem Komplex 1 und der Referenz (Fig. 2) zeigte sich, daß in beiden Fällen nach 38 Tagen erst zwischen 40 und 80 % Wirkstoff freigesetzt werden. Der Komplex 1 war der Referenz hinsichtlich der Verzögerung der Freisetzung überlegen.

Beispiel 2:

| Progesteron-Komplex | |
| --- | --- |
| Komplex 2: | |
| Progesteron | 10 Teile |
| Resomer L 104 | 5 Teile |
| PE 1 | 85 Teile |

Fig. 3 zeigt die Abhängigkeit der prozentualen Freisetzung von Progesteron von der Zeit. Auch Progesteron wird aus einem erfindungsgemäßen Komplex verzögert freigesetzt (Fig. 3). In diesem Falle resultieren nach 24 h etwa 4 % Progesteron im Akzeptormedium. Aufgrund des sich abflachenden Freigabeprofils kann auf eine bis zu 365 Tagen anhaltende Freisetzung des Hormons geschlossen werden. Dies steht im Einklang mit dem langsamen Bioabbau des verwendeten L 104 (Herstellerangabe Boehringer Ingelheim).

Beispiel 3:

| Interleukin-2-Komplex | |
| --- | --- |
| Komplex 3: | |
| Interleukin-2 | 10 Teile |
| Resomer RG 503 H | 10 Teile |
| PE 3 | 80 Teile |

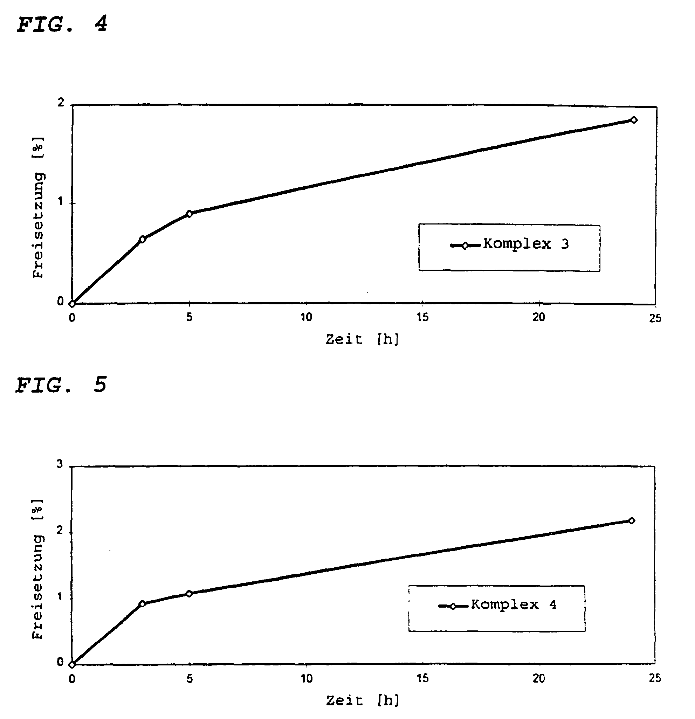

Fig. 4 zeigt die Abhängigkeit der prozentualen Freisetzung von Interleukin-2 von der Zeit. Interleukin-2 wird aus einem erfindungsgemäßen Komplex verzögert freigesetzt (Fig. 4). In diesem Falle resultieren nach 24 h etwa 2 % Interleukin-2 im Akzeptormedium. Aufgrund des sich abflachenden Freigabeprofils kann auf eine lang anhaltende Freisetzung des Proteins geschlossen werden. Dies steht im Einklang mit dem langsamen Bioabbau des verwendeten RG 503 H (Herstellerangabe Boehringer Ingelheim).

Beispiel 4:

| Testosteron-Komplex | |
| --- | --- |
| Komplex 4: | |
| Testosteron | 10 Teile |
| Resomer RG 752 | 15 Teile |
| PE 2 | 75 Teile |

Fig. 5 zeigt die Abhängigkeit der prozentualen Freisetzung von Testosteron von der Zeit. Testosteron wird aus einem erfindungsgemäßen Komplex verzögert freigesetzt (Fig. 5). In diesem Falle resultieren nach 24 h etwa 2 % Testosteron im Akzeptormedium. Aufgrund des sich abflachenden Freigabeprofils kann auf eine lang anhaltende Freisetzung des Hormons geschlossen werden. Dies steht im Einklang mit dem langsamen Bioabbau des verwendeten RG 752 (Herstellerangabe Boehringer Ingelheim).

Untersuchung des Komplexes

Der Anteil PE am Komplex wurde gravimetrisch bestimmt. Dazu wurden die folgenden Komplexe in 0,9 %-iger NaCl-Lösung koaguliert:

| | | |
|---|---|---|
| 1) | Resomer® RG 503 | 10 Teile |
| | PE 2 | 90 Teile, |
| 2) | Resomer® L 104 | 15 Teile |
| | PE 2 | 85 Teile, |
| 3) | Resomer® RG 752 | 20 Teile |
| | PE 2 | 80 Teile, |

Die Masse ($m_0$) an Koagulat wurde erfaßt. Dazu wurden nach 2 h, 24 h und 7 d die Koagulate durch Filtration entnommen und sofort gewogen ($m_{feucht}$). Das feuchte Koagulat wurde im Vakuum bei Raumtemperatur über Silikagel bis zur Massekonstanz getrocknet ($m_{trock}$).

Die Massebilanz des feuchten Koagulats wurde wie folgt berechnet:

Gehalt an Resomer (R%) :

$$R\% = \frac{c \cdot m_0}{m_{feucht}} \cdot 100\%$$

Gehalt an Wasser (W%) :

$$W\% = \frac{m_{feucht} - m_{trock}}{m_{feucht}} \cdot 100\%$$

Gehalt an PE (P%) :

$$P\% = 100\% - R\% - W\%$$

| | |
|---|---|
| $m_o$ | Masse Komplex [g] |
| c | Resomerkonzentration im Koagulat [g/g] |
| $m_{feucht}$ | Masse des feuchten Koagulats [g] |
| $m_{trock}$ | Masse des getrockneten Koagulats [g] |

Aus den Massebilanzen der feuchten Koagulate ergibt sich die Zusammensetzung des trockenen Koagulats aus PE und Resomer, indem R% und P% zu 100 Teilen aufgetragen werden (Fig. 6-8).

Fig. 6 zeigt die Zusammensetzung des trockenen Koagulats 1 in Abhängigkeit von der Zeit.

Fig. 7 zeigt die Zusammensetzung des trockenen Koagulats 2 in Abhängigkeit von der Zeit.

Fig. 8 zeigt die Zusammensetzung des trockenen Koagulats 3 in Abhängigkeit von der Zeit.

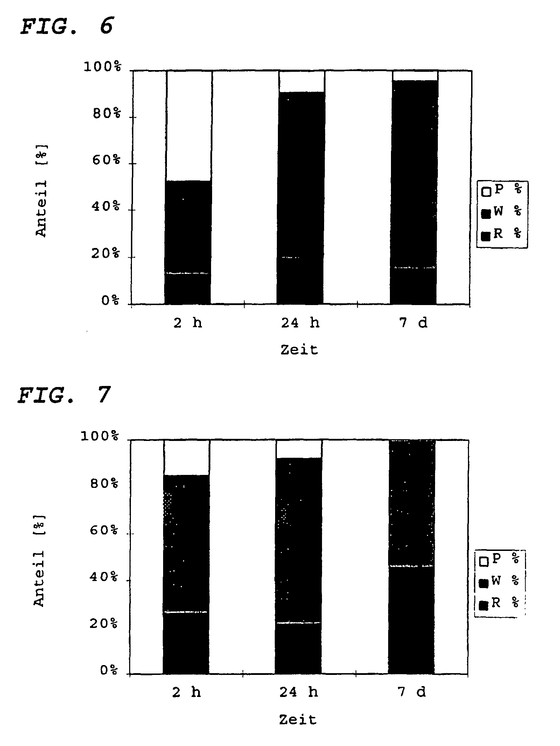

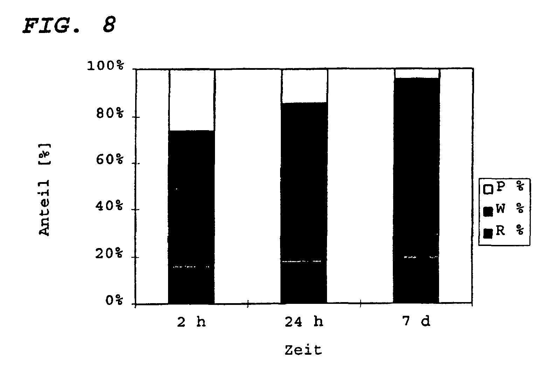

In allen Fällen wurde ein definierter Anteil PE in den getrockneten Koagulaten nachgewiesen (Fig. 6, 7, 8). Der Anteil PE in den getrockneten Koagulaten nimmt bei allen Koagulaten mit der Zeit ab. Ausschwemmen von PE und Bioabbau des Koagulats überlagern sich.

Bezugszeichenliste

1    Komplex
2    Verschlußklemmen
3    Dialyseschlauch
4    Akzeptormedium
5    Magnetrührer
6    Gefäß

**Patentansprüche**

1. Polymerzusammensetzung zur Herstellung eines In-situ-Implantats, bestehend aus einem sterilen injizierbaren wasserunlöslichen Komplex aus einem bioabbaubaren Polymer und einem biokompatiblen endgruppenfunktionalisierten Polyether, wobei das Verhältnis von bioabbaubarem Polymer zu Polyether zwischen 1:100 und 1:2 beträgt und der Komplex unter Einfluß von Körperflüssigkeit koaguliert.

2. Polymerzusammensetzung gemäß Anspruch 1, **dadurch gekennzeichnet, daß** das bioabbaubare Polymer eine Verbindung aus der Gruppe der Poly($\alpha$-hydroxy-ester) oder deren Copolymere ist.

3. Polymerzusammensetzung gemäß Anspruch 1 und 2, **dadurch gekennzeichnet, daß** der biokompatible endgruppenfunktionalisierte Polyether eine Verbindung der allgemeinen Formel I

$$R\text{---}CH_2 \text{---} [CH_2 \text{---} O \text{---} CH_2 \text{---}]_n \text{---} CH_2 \text{---} R \qquad (I)$$

wobei

R für -OH, -CH$_2$OH, -CH$_2$CH$_2$OH steht,
n eine ganze Zahl von 4 bis 12 bedeutet,
und die Molmasse der Verbindung der Formel I 200 bis 600 Masseneinheiten beträgt,

ist.

4. Polymerzusammensetzung gemäß Anspruch 3, **dadurch gekennzeichnet, daß** R für -OH steht.

5. Polymerzusammensetzung gemäß einem der voranstehenden Ansprüche, **dadurch gekennzeichnet, daß** das Verhältnis zwischen bioabbaubarem Polymer und Polyether zwischen 1:6 und 1:3 liegt.

6. Polymerzusammensetzung gemäß einem der voranstehenden Ansprüche, **dadurch gekennzeichnet, daß** mindestens ein biologisch aktiver Stoff aus der Gruppe der Hormone, Immunmodulatoren, Immunsuppressiva, Antibiotka, Zytostatika, Diuretika, Magen-Darm-Mittel, Herz-Kreislauf-Mittel, Antiinflammatoria, Analgetika, Lokalanaesthetika und/oder Neuropharmaka zugesetzt ist, welcher aus dem unter Einfluß der Körperflüssigkeit gebildeten Koagulat freigesetzt wird.

7. Polymerzusammensetzung gemäß einem der voranstehenden Ansprüche, **dadurch gekennzeichnet, daß** man den injizierbaren Komplex sterilisiert.

8. Polymerzusammensetzung gemäß einem der voranstehenden Ansprüche, **dadurch gekennzeichnet, daß** man die Freisetzung des biologisch aktiven Stoffes aus dem gebildeten Koagulat durch die Bestandteile des sterilen injizierbaren wasserunlöslichen Komplexes steuert.


**Claims**

1. Polymer composition for producing an in-situ implant, said polymer composition consisting of a sterile injectable water-insoluble complex formed from a biodegradable polymer and a biocompatible end-group-functionalized polyether, wherein the ratio of biodegradable polymer to polyether is between 1:100 and 1:2 and the complex coagulates under the influence of body fluid.

2. Polymer composition as per claim 1, **characterized in that** the biodegradable polymer is a compound selected from the group of the poly($\alpha$-hydroxy ester)s or their copolymers.

3. Polymer composition as per claim 1 and 2,
**characterized in that** the biocompatible end-group-functionalized polyether is a compound of the general formula I

$$R\text{---}CH_2 [CH_2 \text{---} O \text{---} CH_2 \text{---}]_n \text{---} CH_2 \text{---} R \qquad (I)$$

where

> R represents -OH, -CH$_2$OH, -CH$_2$CH$_2$OH,
> n is an integer from 4 to 12,
> and the molar mass of the compound of formula I is 200 to 600 mass units.

**4.** Polymer composition as per claim 3, **characterized in that** R represents -OH.

**5.** Polymer composition as per any one of the preceding claims, **characterized in that** the ratio between biodegradable polymer and polyether is between 1:6 and 1:3.

**6.** Polymer composition as per any one of the preceding claims, **characterized in that** at least one bioactive substance from the group of the hormones, immunomodulators, immunosuppressants, antibiotics, cytostatics, diuretics, gastrointestinal agents, cardiovascular agents, antiinflammatory agents, analgesics, local anaesthetics and/or neuropharmacological agents has been added that is released from the coagulate formed under the influence of body fluid.

**7.** Polymer composition as per any one of the preceding claims, **characterized in that** the injectable complex is sterilized.

**8.** Polymer composition as per any one of the preceding claims, **characterized in that** the release of the bioactive substance from the coagulate formed is controlled by the constituents of the sterile injectable water-insoluble complex.

**Revendications**

**1.** Composition polymère pour la production d'un implant in situ, constituée d'un complexe stérile injectable, insoluble dans l'eau, à base d'un polymère biodégradable et d'un polyéther biocompatible fonctionnalisé par des groupes terminaux, le rapport entre le polymère biodégradable et le polyéther étant compris entre 1:100 et 1:2 et le complexe coagulant sous l'influence du liquide corporel.

**2.** Composition polymère selon la revendication 1,
**caractérisée en ce que** le polymère biodégradable est un composé parmi le groupe des poly($\alpha$-hydroxyesters) ou de leurs copolymères.

**3.** Composition polymère selon les revendications 1 et 2, **caractérisée en ce que** le polyéther biocompatible fonctionnalisé par des groupes terminaux est un composé de formule générale I

$$R\text{—}CH_2[CH_2\text{—}O\text{—}CH_2\text{—}]_n\text{—}CH_2\text{—}R \qquad (I)$$

dans laquelle

> R désigne -OH, -CH$_2$OH, -CH$_2$CH$_2$OH,
> n représente un entier de 4 à 12,
> et la masse molaire du composé de formule I est de 200 à 600 unités de masse.

**4.** Composition polymère selon la revendication 3, **caractérisée en ce que** R désigne -OH.

**5.** Composition polymère selon l'une quelconque des revendications précédentes, **caractérisée en ce que** le rapport entre le polymère biodégradable et le polyéther est compris entre 1:6 et 1:3.

**6.** Composition polymère selon l'une quelconque des revendications précédentes, **caractérisée en ce qu'**au moins une substance biologiquement active parmi le groupe des hormones, des immunomodulateurs, des immunosuppresseurs, des antibiotiques, des cytostatiques, des diurétiques, des agents gastro-intestinaux, des agents cardiovasculaires, des agents anti-inflammatoires, des analgésiques, des anesthésiques locaux et/ou des agents neuropharmacologiques a été ajoutée et est libérée du coagulat formé sous l'influence du liquide corporel.

7. Composition polymère selon l'une quelconque des revendications précédentes, **caractérisée en ce que** le complexe injectable est stérilisé.

8. Composition polymère selon l'une quelconque des revendications précédentes, **caractérisée en ce que** la libération de la substance biologiquement active du coagulat formé est régulée par les constituants du complexe stérile injectable, insolubles dans l'eau.

FIG. 1

*FIG. 2*

*FIG. 3*

**FIG. 4**

**FIG. 5**

FIG. 6

FIG. 7

FIG. 8